# EUROPEAN PATENT APPLICATION

(11) **EP 3 199 183 A1**
(43) Date of publication of application: **02.08.2017**
(21) Application number: 15843318.5
(22) Date of filing: 07.09.2015
(51) Int. Cl.: A61K 51/00

(54) **RENAL IMAGING AGENT**

(30) Priority: 25.09.2014 JP 2014195802
(71) Applicant: Nihon Medi-Physics Co., Ltd., Tokyo 136-0075 (JP)
(72) Inventor: NAKATA, Norihito, Sodegaura-shi Chiba 299-0266 (JP)
(74) Representative: TBK
(86) International application number: PCT/JP2015/075295
(87) International publication number: WO 2016/047424

(57) **Abstract**

The present invention provides a renal imaging agent comprising a nitroimidazole-type compound or a salt thereof. The renal imaging agent according to the present invention can be used in positron emission tomography.

## Description

### Technical Field

The present invention relates to a renal imaging agent.

### Background Art

The number of patients with renal diseases in Japan tends to increase year by year, and this has a significant impact on the health of the Japanese people. Of the renal diseases, chronic kidney disease (CKD), if worsened, may cause a serious cardiovascular disease, or require dialysis. In recent years, therefore, various measures have been taken to prevent CKD from becoming serious (see Non Patent Literature 1, for example).

The CKD therapeutic guidelines (Non Patent Literature 2) define that CKD is characterized by either one or both of the following symptoms (i) and (ii) lasting for 3 months or longer: (i) the presence of nephropathy is evident from urine abnormality, diagnostic imaging, blood and pathology, and in particular, the presence of 0.15 g/gCr or more of urinary proteins (30 mg/gCr or more of albuminuria) is important; and (ii) GFR (glomerular filtration rate) is less than 60 mL/min/1.73 m². The CKD therapeutic guidelines indicate that the severity of CKD is classified by the GFR and ACR (albumin/creatinine ratio). Non Patent Literature 2 also describes that for diagnosis and determination of therapeutic strategy of CKD, it is recommended that renal biopsy is taken after appropriateness is ascertained with reference to urinalysis findings; and in the case of CKD, it is recommended that abdominal ultrasonography is selected for a diagnosis of a disease showing a morphological change (such as urolithiasis, obstructive uropathy, and cystic kidney disease), and Doppler ultrasonography, MR angiography or CT angiography is selected for evaluation of the presence or absence of, and the degree of, renal artery stenosis, in accordance with renal function.

CKD is characterized by progressive loss of renal function caused by chronic tubulointerstitial disease, including tubular atrophy and interstitial fibrosis. Such changes reduce renal oxygenation, thereby causing fibrillization reactions to be successively initiated and accelerated via various cytokine signaling pathways and cellular signals. Because fibrillization and hypoxia are considered to be primary factors leading to the progression of CKD, an accurate and non-invasive evaluation of these factors is believed to be useful for the treatment of CKD. Non Patent Literature 3 reports that under such a background, detection of an ischemic lesion with CKD was studied by BOLD (blood oxygenation level-dependent)-MRI using fMRI.

Drug-induced nephropathy is nephropathy caused by drugs used for treatment or diagnosis (antibacterial drugs, analgesic drugs, anti-cancer drugs, and contrast media). Many cases of drug-induced nephropathy are reversible; however, a precise early diagnosis is considered to be necessary for avoiding irreversible renal dysfunction, and serum creatinine, urea nitrogen, and general urinalysis are mentioned as essential items for periodic examination of drug-induced nephropathy.

Nuclear medicine examination is known as one technique for examining renal function. The nuclear medicine examination is classified into renography for examining renal dynamics and renal scintigraphy. As radioactive agents used for examining renal dynamics, [¹³¹I] ortho-iodohippurate (¹³¹I-OIH), ^{99m}Tc-MAG3 (mercaptoacetyltriglycine), and ^{99m}Tc-DTPA (diethylenetriaminepentaacetic acid) are known. As a radioactive agent used for renal scintigraphy, ^{99m}Tc-DMSA (dimercaptosuccinic acid) is known. Unlike blood tests and urinalysis, these nuclear medicine examinations have the advantage of evaluating the function of each of the left and right kidneys separately.

Non Patent Literature 1: "kongo-no-jinshikkan-taisaku-no-arikata-ni-tsuite (in Japanese)" ("Regarding How the Future Measures against Renal Diseases Should Be") by "jinshikkann-taisaku-kentou-kai (in Japanese)" (the Committee on the Study of Measures against Renal Diseases) (March, 2008)
Non Patent Literature 2: Evidence-based CKD Therapeutic Guidelines 2013
Non Patent Literature 3: Journal of the American Society of Nephrology, 2011, 22(8), pp.1429-34
Non Patent Literature 4: Kidney International, 2008, 74, pp. 867-872

### Summary of Invention

The conventional methods for examining renal diseases, however, have the following problems. As described in Non Patent Literature 2, in the case of CKD, there is a report on the retrospective study that the introduction of a specialist after the stage G3 section (stage G4 at the latest) slowed down the rate of deterioration of renal function, achieving a delay in the time when dialysis is to be introduced. One possible reason for this may be drug adjustment by the specialist, although no reliable evidence has been obtained.

Furthermore, renal biopsy is considered to be useful for determination of a therapeutic strategy or long-term prognosis of CKD, whereas renal function and ischemia cannot be found from renal biopsy.

The conventional nuclear medicine diagnostic methods have not been sufficiently verified in terms of the reliability of measured values for renal function, and their utility for clinical tests remains problematic.

As described in Non Patent Literature 4, it is known that a hypoxic state within kidney worsens nephropathy; however, no established technique is known that allows detection of the hypoxic state within kidney.

In the case of a drug-induced nephropathy that irreversibly occurs, it is too late when the abnormality is detected in urinalysis or a blood test, and even the cessation of medication may not recover renal dysfunction.

The present invention is made in view of the foregoing circumstances, and an object of the present invention is to provide a novel renal imaging agent that can non-invasively visualize a lesion part based on the renal environment.

The present inventors have newly found that a renal lesion can be visualized non-invasively with the nuclear medicine examination using a radioactive fluorine (¹⁸F) - labeled specific nitroimidazole-based compound. The nitroimidazole-based compound accumulates specifically in a hypoxic region. Thus, early detection, early treatment, prognosis and prediction, and therapeutic effect assessment of renal diseases are expected to be possible by detecting the spread of a hypoxic state within kidney and quantitatively evaluating the hypoxic state to evaluate degree of fibrillization of the kidney.

In summary, according to the present invention, there is provided a renal imaging agent comprising a nitroimidazole-based compound represented by the general formula (1) shown below, or a salt thereof.

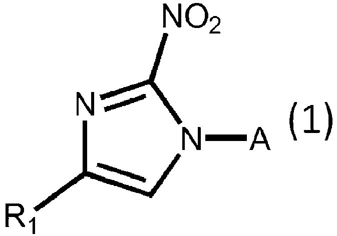

In the general formula (1) shown above, R₁ is hydrogen or a hydroxymethyl group. A is any one of the groups (I) to (IV) shown below. 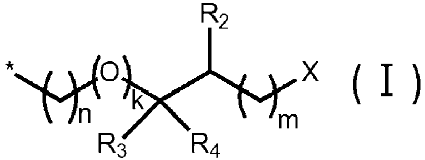 In the group represented by (I), R₂ is hydrogen or a hydroxy group; R₃ is hydrogen or a hydroxymethyl group; R₄ is a hydroxy group or a hydroxymethyl group; k is 0 or 1; m is 0 or 1; n is 0, 1 or 2; and X is a radioactive fluorine. 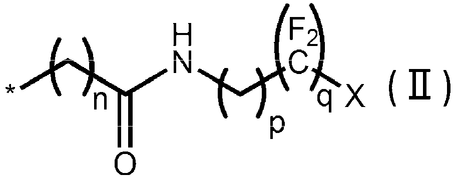 In the group represented by (II), n is 0, 1 or 2; p is 1 or 2; q is 0, 1 or 2; and X is a radioactive fluorine. 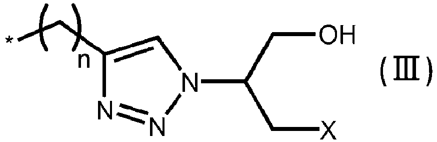 In the group represented by (III), n is 0, 1 or 2; and X is a radioactive fluorine. 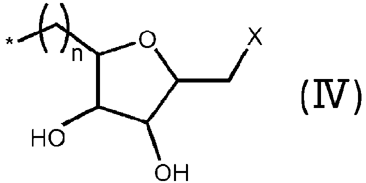 In the group represented by (IV), n is 0, 1 or 2; and X is a radioactive fluorine.

According to the present invention, a renal imaging agent can be provided that allows a lesion part to be non-invasively visualized based on the renal environment.

### Brief Description of Drawings

The foregoing object and other objects, features and advantages will become more apparent from the preferred embodiments described below, as well as the following accompanying drawings.

Figure 1 shows PET images (MIP images) with ¹⁸F-HIC101, wherein (a) is an image of a CKD model, and (b) is an image of a healthy model.
Figure 2 is a diagram showing the results of in vivo distribution 100 minutes after the administration of ¹⁸F-HIC101.
Figure 3 is a picture showing a comparison between the level of expression of HIF-1α and the localization of ¹⁸F-HIC101 within renal tissue.
Figure 4 shows PET images (MIP images) with ¹⁸F-FMISO, wherein (a) is an image of a CKD model, and (b) is an image of a healthy model.

### Description of Embodiments

In the present invention, the "radioactive fluorine" refers to a radioactive isotope of fluorine, i.e., fluorine-18 (¹⁸F).

In the present invention, the "salt" may be any pharmaceutically acceptable salt, and includes, for example, those derived from inorganic acids such as hydrochloric acid, hydrobromic acid, sulfuric acid, nitric acid, and phosphoric acid; or those derived from organic salts such as acetic acid, trifluoroacetic acid, maleic acid, succinic acid, mandelic acid, fumaric acid, malonic acid, pyruvic acid, oxalic acid, glycolic acid, salicylic acid, pyranosidyl acids (such as glucuronic acid and galacturonic acid), α-hydroxy acids (such as citric acid and tartaric acid), amino acids (such as aspartic acid and glutamic acid), aromatic acids (such as benzoic acid and cinnamic acid), and sulfonic acids (such as p-toluenesulfonic acid and ethanesulfonic acid).

In the present invention, the "nitroimidazole-based compound" refers to one represented by the general formula (1) shown above, and includes, for example, the followings:
2-[¹⁸F]fluoromethyl-2-((2-nitro-1H-imidazol-1-yl)methyl]-1,3-propanediol (¹⁸F-HIC101: a compound wherein A is a group represented by (I), R₁ and R₂ are hydrogen, R₃ and R₄ are hydroxymethyl groups, k is 0, m is 0, and n is 1);
2-[¹⁸F]fluoromethyl-2-((4-hydroxymethyl-2-nitro-1H-imidazol-1-yl)methyl)-1,3-propanediol (a compound wherein A is a group represented by (I), R₁, R₃, and R₄ are hydroxymethyl groups, R₂ is hydrogen, k is 0, m is 0, and n is 1);
2-[¹⁸F]fluoromethyl-2-(2-(2-nitro-1H-imidazol-1-yl)ethyl)-1,3-propanediol (a compound wherein A is a group represented by (I), R₁ and R₂ are hydrogen, R₃ and R₄ are hydroxymethyl groups, k is 0, m is 0, and n is 2);
1-[¹⁸F]fluoro-3-(2-nitro-1H-imidazol-1-yl)-2-propanol (¹⁸F-FMISO: a compound wherein A is a group represented by (I), R₁, R₂, and R₃ are hydrogen, R₄ is a hydroxy group, k is 0, m is 0, and n is 1);
1-[¹⁸F]fluoro-4-(2-nitro-1H-imidazol-1-yl)-2,3-butanediol (¹⁸F-FETNIM: a compound wherein A is a group represented by (I), R₁ and R₃ are hydrogen, R₂ and R₄ are hydroxy groups, k is 0, and m and n are 1);
3-[¹⁸F]fluoro-2-((2-nitro-1H-imidazol-1-yl)methoxy)-1-propanol (¹⁸F-FRP-170: a compound wherein A is a group represented by (I), R₁, R₂, and R₃ are hydrogen, R₄ is a hydroxymethyl group, k is 1, m is 0, and n is 1);
N-(2-[¹⁸F]fluoroethyl)-2-nitro-1H-imidazole-1-acetamide (¹⁸F-FETA: a compound wherein A is a group represented by (II), R₁ is hydrogen, n is 1, p is 2, and q is 0);
2-nitro-N-(2,2,3,3,3-[¹⁸F]pentafluoropropyl)-1H-imidazole-1-acetamide (¹⁸F-EF5: a compound wherein A is a group represented by (II), R₁ is hydrogen, n is 1, p is 1, and q is 2);
(3-[¹⁸F]fluoro-2-(4-((2-nitro-1H-imidazol-1-yl)methyl)-1H-1,2,3-triazol-1-yl)-1-propanol (¹⁸F-HX4: a compound wherein A is a group represented by (III), R₁ is hydrogen, and n is 1); or
1-(5-deoxy-5-[¹⁸F]fluoro-α-D-arabinofuranosyl)-2-nitro-1H-imidazole (¹⁸F-FAZA: a compound wherein A is a group represented by (IV), R₁ is hydrogen, and n is 0).
In (I) to (IV), * (asterisk) represents a bonding site.

From the standpoint of increasing the uptake in a renal lesion, a nitroimidazole-based compound having lipophilicity lower than that of ¹⁸F-FMISO is preferred, and specifically, a nitroimidazole-based compound having an octanol/water partition coefficient (logP) at 25°C lower than the logP of ¹⁸F-FMISO. A nitroimidazole-based compound having a logP of -0.4 or less is more preferred, and a nitroimidazole-based compound having a logP in the range from -2 to -0.6 is even more preferred. In the structure of the nitroimidazole-based compound, R₁ is preferably hydrogen in the general formula (1).

In the nitroimidazole-based compounds represented by the general formula (1) wherein A is (I), R₂ in (I) is preferably hydrogen from the standpoint of increasing the uptake in a renal lesion. R₄ is preferably a hydroxymethyl group; m is preferably 0; and n is preferably 1. More preferably, R₃ is a hydroxymethyl group, and k is 0. These nitroimidazole-based compounds can be synthesized based on WO2013/042668; "Production and Quality Control of Radioactive Agents for PET - Handbook of Synthesis and Clinical Use" (edited by PET Chemistry Workshop) - 4th edition (revised version in 2011); J. Nucl. Med, 2001, 42, pp. 1397-1404; Annals of Nuclear Medicine, 2007, 21, pp. 101-107; and other known information.

In the nitroimidazole-based compounds represented by the general formula (1) wherein A is (II), n is preferably 1 from the standpoint of increasing the uptake in a renal lesion. Furthermore, when p is 2, q is preferably 0, and when p is 1, q is preferably 2. These nitroimidazole-based compounds can be synthesized based on, for example, British Journal of Cancer, 2004, 90, pp. 2232-2242; Applied Radiation and Isotopes, 2001, 54, pp. 73-80; and other known information.

In the nitroimidazole-based compounds represented by the general formula (1) wherein A is (III), n is preferably 1 from the standpoint of increasing the uptake in a renal lesion. These nitroimidazole-based compounds can be synthesized based on WO2008/124651 and other known information.

In the nitroimidazole-based compounds represented by the general formula (1) wherein A is (IV), n is preferably 0 from the standpoint of increasing the uptake in a renal lesion. These nitroimidazole-based compounds can be synthesized based on "Production and Quality Control of Radioactive Agents for PET - Handbook of Synthesis and Clinical Use" (edited by PET Chemistry Workshop) - 4th edition (revised version in 2011); and other known information.

The renal imaging agent according to the present invention can be defined as a formulation containing the nitroimidazole-based compound represented by the general formula (1) shown above or a salt thereof in a form suitable for administration into living body. The renal imaging agent according to the present invention is preferably in a form that is to be administered parenterally, i.e., by injection, and is more preferably an aqueous solution. Such a composition may contain additional components such as a pH adjuster, a pharmaceutically acceptable solubilizer, a stabilizer, or an antioxidant, as required.

When the renal imaging agent according to the present invention is introduced into living body, the nitroimidazole-based compound represented by the general formula (1) shown above accumulates in hypoxic renal tissue. This allows radiation to be detected non-invasively from the outside of the living body by using positron emission tomography (PET), thereby enabling the spread or degree of the renal lesion to be imaged. Thus, with regard to various kidney diseases, the renal imaging agent of the present invention can provide renal function information that cannot be obtained by the conventional examination methods, so as to realize early detection, early treatment, prognosis and prediction, and therapeutic effect assessment of renal diseases.

For example, in the case of CKD, the timing of dialysis initiation can be deferred by quantifying the uptake of radioactivity in the renal cortex using the renal imaging agent according to the present invention, thereby appropriately understanding the degree of progression of fibrillization and optimizing the drug therapy. Furthermore, because information on renal function and ischemia can be obtained with the renal imaging agent of the present invention, the use thereof with kidney biopsy in a complementary manner allows more accurate understanding of pathology of CKD as well as prognosis and prediction of CKD.

Furthermore, during drug therapy with anticancer agents or the like, renal dysfunction can be detected earlier than a change in blood or urine, by monitoring renal function using the renal imaging agent according to the present invention. Thus, irreversible drug-induced nephropathy can be avoided by stopping the drug administration or changing the drug.

### Examples

Hereinafter, the present invention will be explained in more detail by describing working examples; however, the present invention is not limited to the contents of these examples.

The compounds used in the present examples are defined as follows. Both compounds were synthesized in accordance with the method described in WO2013/042668.

¹⁸F-HIC101 : 2-[¹⁸F]fluoromethyl-2-((2-nitro-1H-imidazol-1-yl)methyl)-1,3-propanediol (compound 1 in the EXAMPLES of WO2013/042668)
¹⁸F-FMISO: 1-[¹⁸F]fluoro-3-(2-nitro-1H-imidazol-1-yl)-2-propanol (¹⁸F-fluoromisonidazole)

### Example 1: Preparation of CKD model animals [1]

Adriamycin (from Wako Pure Chemical Industries, Ltd., 7.5 mg/kg) was administered to 13 Lewis rats (male, 8-week-old, available from Japan SLC, Inc.) via the tail vein, and urinary protein was measured in accordance with the Bradford method on day 13 after the administration, for 11 cases excluding two dead cases. Of these, four cases having a high urinary protein level were selected as CKD model animals, and used in the below-described examples on day 14 after the administration of adriamycin. Table 1 shows the conditions of the four cases.

As healthy models, four cases prepared by administering an equivalent amount of physiological saline instead of adriamycin were used.

The amount of FABP-4 in the urine of each model was measured using an ELISA kit (from R&D Systems) which quantifies L-FABP present in murine or rat samples by sandwich method. Measurement of urinary creatinine was also performed using a kit (from Cayman Chemical) which utilizes Jaffe reaction, in order to correct the influence of concentration and dilution of urinary components due to living activity.

**Table 1**

| | ¹⁸F-FMISO-Treated Group | | ¹⁸F-HIC101-Treated Group | |
|---|---|---|---|---|
| | Healthy Model Group | CKD Model Group | Healthy Model Group | CKD Model Group |
| Body Weight (g) | 268±11.3 | 203±8.25* | 261±9.19 | 210±13.5* |
| Urinary Protein (g/Cr mmol) | 0.07±0.01 | 3.61±1.06* | 0.01±0.01 | 5.72±2.88* |
| L-FABP (µg/Cr mmol) | 0.04±0.01 | 1.05±0.27* | 0.04±0.04 | 1.24±0.77* |

| | | | | |
|---|---|---|---|---|
| *p<0.05 (CKD Models vs. Healthy Models) | | | | |

Table 1 shows the mean ± standard deviation for each of the four cases. In the CKD model groups, there was no significant difference in urinary protein level and L-FABP level between the ¹⁸F-FMISO-treated group and the ¹⁸F-HIC101-treated group.

### Example 2: PET imaging [1]

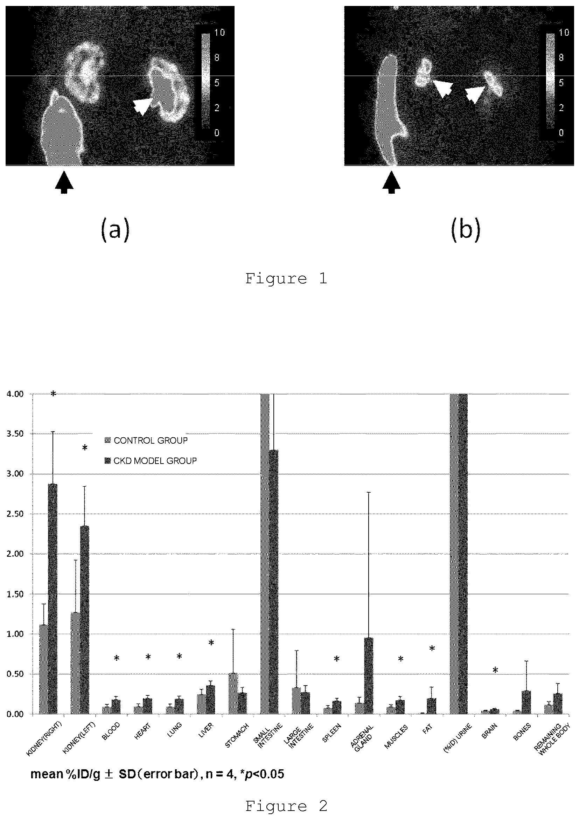

¹⁸F-HIC101 (radiochemical purity: 84.2%) was administered to the four CKD models prepared in Example 1 at 18.6 ± 0.9 MBq/rat and to four healthy models at 17.0 ± 2.7 MBq/rat, and after 80 minutes from the administration, static imaging was performed using a PET system for animals (explore Vista from GE). The collection conditions were 10 minutes at an energy window of 250 to 700 keV. The collected data were reconstructed and imaged using the 3D-OSEM method. From the images, the average of maximum values of SUV (standardized uptake value) of the kidneys (the region of interest (ROI) was set excluding the renal pelvis) and the average value of SUV of normal tissue in each slice were measured. Based on these values, the lesion-to-normal tissue ratio and the normal kidney-to-normal tissue ratio were used for evaluation. Student's t-test was used for statistical analysis of the measured results. The results are shown in Figure 1 and Table 2.

**Table 2**

| | | CKD Model Group | Healthy Model Group |
|---|---|---|---|
| Renal Tissue SUV Maximum Value | Right | 5.17±0.70* | 1.42±0.43 |
| | Left | 5.25±0.55* | 1.37±0.28 |
| Normal Tissue SUV Average Value | | 0.46±0.04^{$} | 0.37±0.06 |
| Renal Tissue (Lesion or Normal)-to-Normal Tissue Ratio | Right | 11.33±1.57* | 3.77±0.66 |
| | Left | 11.59±1.88* | 3.69±0.37 |

| | | | |
|---|---|---|---|
| (Mean ± Standard Deviation), *p<0.001, $p=0.045, n=4, student's t-test | | | |

Figure 1 shows MIP images obtained by image processing using maximum intensity projection. Figure 1(a) shows a CKD model, and Figure 1(b) shows a healthy model. In Figure 1, the intestinal tract is indicated by the black arrows, and the renal pelvis is indicated by the white arrows. The image analysis showed that the SUV maximum value of renal tissue (excluding the renal pelvis) in the CKD model was significantly higher than that in the healthy model (p<0.001 for both left and right kidneys), and the lesion-to-normal tissue ratio was also significantly higher in the CKD model (p<0.001 for both left and right kidneys). In the example shown in Figure 1, the SUV maximum value of renal tissue was 10. The SUV average value of normal tissue was also found to be significantly higher in the CKD model (p=0.045).

### Example 3: Experiment of in vivo distribution [1]

After the completion of the PET imaging in Example 2, these rats were placed under anesthesia until 100 minutes after the administration, and were sacrificed by exsanguination. Then, the left and right kidneys, blood, brain, lung, heart, liver, spleen, stomach, small intestine, large intestine, adrenal gland, muscles, bones, fat around the kidneys, urine, and the remaining whole body were extracted, and weights and amounts of radioactivity were measured. Student's t-test was used for statistical analysis of the results. The results are shown in Figure 2 and Table 3.

| Table 3 | CKD Model | Healthy Model | | CKD Model | Healthy Model |
|---|---|---|---|---|---|
| Kidney (Right) | 2.60±0.58* | 1.01±0.24 | Liver | 0.34±0.05* | 0.23±0.06 |
| Kidney (Left) | 2.11±0.44* | 1.17±0.61 | Small Intestine | 0.25±0.82 | 4.50±0.70 |
| Blood | 0.16±0.04* | 0.08±0.03 | Large Intestine | 3.17±0.08 | 0.32±0.44 |
| Heart | 0.18±0.04* | 0.09±0.03 | Muscles | 0.26±0.04 | 0.09±0.03 |
| Lung | 0.18±0.05* | 0.08±0.03 | Urine (%ID) | 29.9±14.9 | 34.4±23.3 |

| | | | | | |
|---|---|---|---|---|---|
| Mean ± Standard Deviation, *p<0.05, n=4 student's t-test | | | | | |

In Figure 2, for each organ, the left bar represents the healthy model group, and the right bar represents the CKD model group. The uptake in the left and right kidneys was significantly higher in the CKD model group than in the healthy model group. Furthermore, the uptake in the blood, heart, lung, liver, and muscles, which are major tissues, was significantly higher in the CKD model group. The absence of a significant difference in the small intestine, large intestine, and urine is believed to be due to individual differences in bile excretion rate and urine excretion.

### Example 4: Evaluation of localization of uptake within the kidneys

The renal tissue obtained in Example 3 was divided into halves after the measurement of the amounts of radioactivity, one of the halves was embedded in O.C.T. Compound (from Sakura Fineteck Japan) to prepare fresh frozen sections (thickness: 10 µm) using a cryostat (model: CM3050 from Leica), and autoradiography was performed using these frozen sections. These renal tissue sections were exposed to an imaging plate for 8 to 10 hours, and then imaged using a bio-imaging analyzer (model: BAS-2500 from Fujifilm Corporation).

Then, immunohistochemistry (LSAB method) was performed using the same section after the radioactive decay. After the fixation and activation treatment of the renal tissue section, anti-rat HIF-1α mouse monoclonal antibody (available from GeneTex, 100-fold dilution) used as the primary antibody and anti-mouse IgG antibody (available from DAKO) used as the secondary antibody were reacted with the renal tissue section. Then, using HRP-labeled streptavidin (from DAKO) that reacts with the secondary antibody, the HRP activity was detected by the color reaction with DAB (3,3'-diaminobenzidine) as the substrate to identify the sites of expression of HIF-1α in the renal tissue section. Using, as a negative control, a proximate section which is a consecutively sectioned single thin sheet, the same experiment as that described above was performed following the same procedure except that the primary antibody was not used for the reaction. As a result, it was confirmed that there was no non-specific reaction to the renal tissue section due to components other than the primary antibody. Using a microscope system (model: BZ-9000 from KEYENCE), the whole of the specimen image obtained by the immunohistochemical staining was acquired. The image was subjected to image processing in which DAB-positive sites were extracted from the image using ImageJ, and then pseudo-colored.

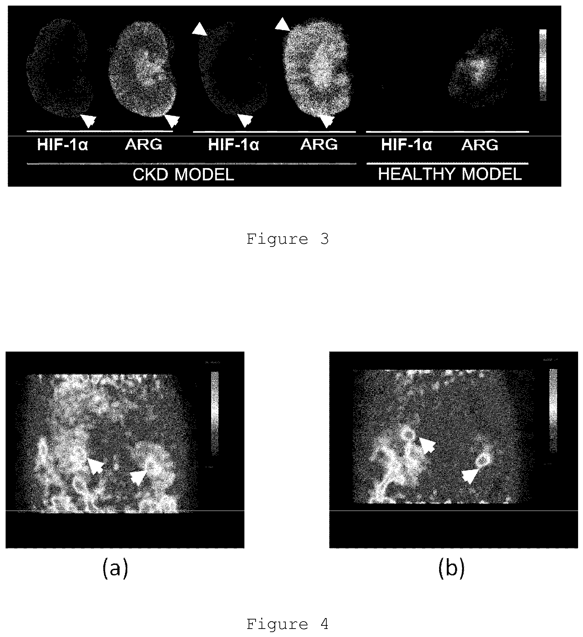

The results are shown in Figure 3. The expression of HIF-1α within the renal tissue was confirmed in two CKD models (SUV maximum value: (left) 4.30, (right) 5.12) and one healthy model (SUV maximum value: 1.06). As a result, high expression of HIF-1α was visually observed in the renal cortex of the CKD models. From a comparison with the localization of ¹⁸F-HIC101 by autoradiography, the sites indicated by the white arrows in Figure 3 corresponded to the sites of expression of HIF-1α. In Figure 3, ARG is an abbreviation for autoradiography. The area showing the uptake in the autoradiogram of the untreated corresponds to the renal pelvis.

### Example 5: PET imaging [2]

¹⁸F-FMISO (radiochemical purity: 96% or more) was administered to the four CKD models prepared in Example 1 at 18.7 ± 1.1 MBq/rat and to four healthy models at 19.5 ± 0.69 MBq/rat, and after 80 minutes from the administration, static imaging was performed using a PET system for animals (explore Vista from GE). One case each from each of the groups was placed under anesthesia again after the PET imaging, and imaging was performed after 180 minutes from the administration. The collection conditions were 10 minutes at an energy window of 250 to 700 keV, and the collected data were reconstructed and imaged using the 3D-OSEM method. From the images, the average of maximum values of SUV of the kidneys (the region of interest (ROI) was set excluding the renal pelvis) and the average value of SUV of normal tissue in each slice were measured. Based on these values, the lesion-to-normal tissue ratio and the normal kidney-to-normal tissue ratio were used for evaluation. Student's t-test was used for statistical analysis of the measured results. The results are shown in Figure 4 and Table 4.

**Table 4**

| | | After 180 min from Administration | | After 80 min from Administration | |
|---|---|---|---|---|---|
| | | CKD Model | Healthy Model | CKD Model | Healthy Model |
| Renal Tissue SUV Maximum Value | Right | 2.51 | 1.63 | 2.08±0.22* | 1.68±0.14 |
| | Left | 2.77 | 1.67 | 1.97±0.15* | 1.74±0.08 |
| Normal Tissue SUV Average Value | | 0.87 | 0.72 | 0.94±0.11 | 0.89±0.03 |
| Renal Tissue (lesion or normal)-to-Normal Tissue Ratio | Right | 2.87 | 2.26 | 2.22±0.10* | 1.89±0.13 |
| | Left | 3.17 | 2.31 | 2.11±0.14 | 1.95±0.05 |

| | | | | | |
|---|---|---|---|---|---|
| (Mean ± Standard Deviation), *p<0.05 (CKD Models vs. Healthy Models) | | | | | |

As a result of analysis of the PET images after 80 minutes from the administration, the SUV maximum value of renal tissue (excluding the renal pelvis) in the CKD models was found to be significantly higher than that in the healthy models (p<0.05 for both left and right kidneys); however, a significant difference was not observed in the lesion-to-normal tissue ratio in the left kidney (right: p<0.01, left: p=0.08).

A comparison of the PET images after 180 minutes from the administration with the PET images after 80 minutes from the administration showed a tendency toward a higher uptake in the renal cortex than in the renal pelvis. It was visually confirmed that there was no difference in background between the CKD models and healthy models, and this was the same as that after 80 minutes from the administration. Results of the ROI analysis showed a tendency for the SUV maximum value to increase more than that after 80 minutes from the administration, and for the lesion-to-normal tissue ratio to also increase.

Figure 4 shows MIP images taken after 180 minutes from the administration, obtained by image processing using maximum intensity projection. Figure 4(a) shows a CKD model, and Figure 4(b) shows a healthy model. In Figure 4, the renal pelvis is indicated by the white arrows.

### Example 6: Experiment of in vivo distribution [2]

Three cases from each of the groups that underwent the PET imaging after 80 minutes from the administration in Example 5, as well as one case from each of the groups that had not undergone PET imaging, were placed under anesthesia until 100 minutes after the administration, and were sacrificed by exsanguination. Then, the left and right kidneys, blood, brain, lung, heart, liver, spleen, stomach, small intestine, large intestine, adrenal gland, muscles, bones, fat around the kidneys, urine, and the remaining whole body were extracted, and weights and amounts of radioactivity were measured. Student's t-test was used for statistical analysis of the results. The results are shown in Table 5.

| Table 5 | CKD Model | Healthy Model | | CKD Model | Healthy Model |
|---|---|---|---|---|---|
| Kidney (Right) | 1.09±0.29 | 0.55±0.03 | Liver | 0.89±0.28 | 0.50±0.04 |
| Kidney (Left) | 1.08±0.28 | 0.53±0.03 | Small Intestine | 1.54±0.67 | 0.74±0.11 |
| Blood | 0.44±0.02 | 0.30±0.02 | Large Intestine | 1.14±0.10 | 1.52±0.15 |
| Heart | 0.53±0.01 | 0.34±0.02 | Muscles | 0.47±0.02 | 0.30±0.02 |
| Lung | 0.48±0.02 | 0.31±0.02 | Urine (%ID) | 2.60±2.16 | 9.50±1.75 |

| | | | | | |
|---|---|---|---|---|---|
| Mean ± Standard Deviation | | | | | |

The foregoing results confirmed that both ¹⁸F-HIC101 and ¹⁸F-FMISO are uptaken into the renal tissue excluding the renal pelvis in the CKD models significantly, compared with the healthy models. Furthermore, from the comparison of their uptake in the CKD models, the uptake of ¹⁸F-HIC101 was confirmed to be higher than the uptake of ¹⁸F-FMISO. These results indicate that imidazole-based compounds, in particular, ¹⁸F-HIC101, are useful as renal imaging agents.

This application claims a priority from the Japanese Patent Application No. 2014-195802 filed on September 25, 2014, the disclosure of which is incorporated herein in its entirety.

## Claims

1. A renal imaging agent comprising a nitroimidazole-based compound represented by the general formula (1) shown below, or a salt thereof. 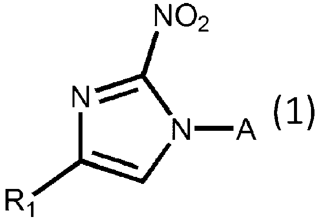 wherein R₁ is hydrogen or a hydroxymethyl group, and A is any one of the groups (I) to (IV) shown below: 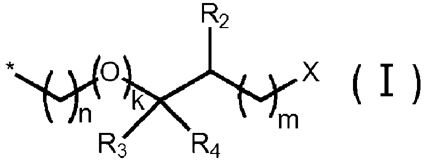 wherein R₂ is hydrogen or a hydroxy group, R₃ is hydrogen or a hydroxymethyl group, R₄ is a hydroxy group or a hydroxymethyl group, k is 0 or 1, m is 0 or 1, n is 0, 1 or 2, and X is radioactive fluorine; 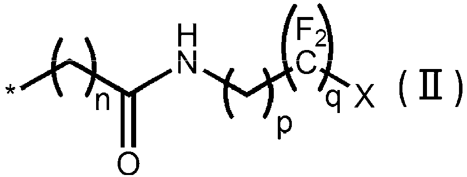 wherein n is 0, 1 or 2, p is 1 or 2, q is 0, 1 or 2, and X is a radioactive fluorine; 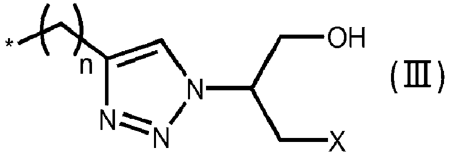 wherein n is 0, 1 or 2, and X is a radioactive fluorine; and

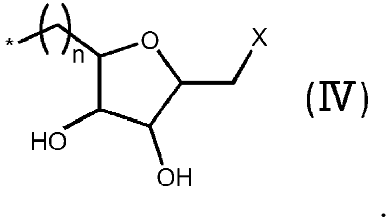

2. The renal imaging agent according to claim 1, wherein the nitroimidazole-based compound is
2-[¹⁸F]fluoromethyl-2-((2-nitro-1H-imidazol-1-yl)methyl)-1,3-propanediol;
2-[¹⁸F]fluoromethyl-2-((4-hydroxymethyl-2-nitro-1H-imidazol-1-yl)methyl)-1,3-propanediol;
2-[¹⁸F]fluoromethyl-2-(2-(2-nitro-1H-imidazol-1-yl)ethyl)-1,3-propanediol;
1-[¹⁸F]fluoro-3-(2-nitro-1H-imidazol-1-yl)-2-propanol;
1-[¹⁸F]fluoro-4-(2-nitro-1H-imidazol-1-yl)-2,3-butanediol;
3-[¹⁸F]fluoro-2-((2-nitro-1H-imidazol-1-yl)methoxy)-1-propanol;
N-(2-[¹⁸F]fluoroethyl)-2-nitro-1H-imidazole-1-acetamide;
2-nitro-N-(2,2,3,3,3-[¹⁸F]pentafluoropropyl)-1H-imidazole-1-acetamide;
(3-[¹⁸F]fluoro-2-(4-((2-nitro-1H-imidazol-1-yl)methyl)-1H-1,2,3-triazol-1-yl)-1-propanol; or
1-(5-deoxy-5-[¹⁸F]fluoro-α-D-arabinofuranosyl)-2-nitro-1H-imidazole.

3. The renal imaging agent according to claim 1, wherein the nitroimidazole-based compound is a compound having a logP at 25°C lower than a logP of 1-[¹⁸F]fluoro-3-(2-nitro-1H-imidazol-1-yl)-2-propanol.

4. The renal imaging agent according to any one of claims 1 to 3, which is used for positron emission tomography.
